# EUROPEAN PATENT APPLICATION

(11) **EP 3 108 877 A1**
(43) Date of publication of application: **28.12.2016**
(21) Application number: 16173700.2
(22) Date of filing: 09.06.2016
(51) Int. Cl.: A61K 9/00, A61K 9/06, A61K 36/28, A61K 36/534, A61K 36/73, A61P 17/02

(54) **TOPICAL COMPOSITION COMPRISING NATURAL INGREDIENTS FOR BRUISING OR HEMATOMA HEALING ON SKIN AND USE THEREOF**

(30) Priority: 24.06.2015 US 201514748347
(71) Applicant: Amphora Holdings, Inc., Fullerton, CA 92835 (US)
(72) Inventor: WURTS, Elmer David, Brea, CA California 92822 (US)
(74) Representative: Gallego Jiménez, José Fernando

(57) **Abstract**

A topical composition is provided that comprises an organic liquid with enzymes, an anti-inflammatory and a topical analgesic for healing bruises or hematomas caused by contusions or lesions on tissues or resulting from illnesses. The organic liquid with enzymes can include Pyrus Malus (apple), the anti-inflammatory can include sesquiterpene lactone (arnica), and the topical analgesic can included menthol, chamomile, ginger, or Aloe Vera. The topical composition can be used to alleviate the hematoma or bruise produced by illness, particularly to disaggregate the hematoma, lower the amount of discoloration of the hematoma or bruise, decrease the amount of pain associated with the hematoma or bruise, and/or decrease inflammation associated with the hematoma or bruise.

## Description

### Field of the Invention

Present invention is directed to a topical composition comprising natural origin ingredients, such as active ingredients and to the use thereof for the healing of bruises or hematomas on the skin, as well as pain, inflammation and change of coloring on the skin which is associated with these. Particularly, the topical composition of the invention allows for the healing of hematomas or bruises which are generated by contusions or lesions to tissue or even as a result of illnesses whose symptomatology produces hematomas or bruising.

### Background of the Invention

This section provides background information related to the present disclosure which is not necessarily prior art.

The hematomas or "bruises" are spots or marks which appear on the skin (echymossis) as a result of blood accumulation which remains trapped under the surface of the skin. This blood accumulation is caused by some lesion or contusion which derives in the rupture of small capillary vessels without the skin actually breaking.

The hematomas may present themselves in different parts of the body, being classified as: subcutaneous (found under the skin); intramuscular (found in the underlying muscle, generally on the protruding part); and periosteal (found on some bone).

The hematomas are generally accompanied by pain, inflammation and change of coloring to the skin. Regarding the change of coloring, the skin initially presents a pinkish color, which changes to a bluish coloring and gradually turns into a yellowish-greenish coloring with the passage of days until eventually reaching its normal coloring.

The time of duration of a hematoma on the skin is variable; the skin may be spotted for approximately 02 weeks or even yet, for several months.

Traditionally, ice is applied to the affected area in order to heal the hematomas. In case it was necessary, creams or ointments could be applied over the hematoma and analgesic may even be administered if the presence of pain ends up being uncomfortable for the person.

Another determining factor for the appearance of hematomas is in relation with certain types of diseases which produce these types of alterations to the skin. Among the illnesses which are characterized by the appearance of hematomas as part of their symptoms are: purpura, Henoch-Schonlein purpura, Senile Purpura or Bateman purpura, capillaritis, Von Willebrand disease, hemophilia, and thrombocytopenia among others.

In prior art various patent applications are known for the treatment of hematomas: CN103099754 is directed at a cream with regenerative effects and relief from mosquito bites, bruises and inflammation. The composition comprises paraffin, Vaseline, white mineral oil, wintergreen oil, essence of lavender, lanoline, menthol and shikone extract. US20110293696 describes a composition for the treatment of bruises which includes an antioxidant, citrus flavonoids together with excipients or pharmaceutically acceptable fillers.

WO201107794 teaches a composition comprising zeolite or sericite for treating among others, bruises.

US20100016446 discloses topical creams which are water based, comprising nitroglycerine, penetration ameliorative, aqueous dissolvents and pH adjusters. The cream may be used for the healing of bruises.

CA2620242 describes topical preparations for the healing and relief of bruises. These preparations comprise active ingredients such as arnica, medicinal solution of witch hazel and menthol.

CA2555617 teaches of a cream or balm comprising vegetable oil, natural waxes, Taraxacam Officianalis, Plectranthus australis, Calendula officinale and Benzoin and which ends up being useful in combating muscular pain, pain and bruising on the skin as well as for bones.

US20070275105 discloses a composition to diminish bruising comprising glycerin, evening primrose oil, fragrance and vitamins A, C, D, E and K.

US20060257509 relates to a topical composition comprising Rosmarinus officinalis, Centella, Echinacea or Alpinia, at least one DNA repair enzyme and pharmaceutically or cosmetically acceptable vehicles, useful for the treatment of bruises, among others. WO200632091 teaches herbal compositions comprising arnica, medicinal solution of witch hazel, Comfrey, calendula, St. John's wort and lavender for the treatment of skin wounds, such as bruises.

US20040131579 describes a topical composition which comprises a fitoextract and a vehicle which allows shortening the duration of bruises in premenopausal or menopausal women.

EP1277474 discloses topical compositions comprising arnica, Ruscus and mint extracts for the treatment of bruises.

Despite the compositions which are already known in the state of the art for the relief of bruising on the skin, a need exists for new compositions which provide for fast and efficient healing, particularly for those which are generated by contusions or lesions to the tissue or as a result of illnesses.

### Brief Description of the Invention

The present invention provides a topical composition comprising active ingredients from natural origins for the healing of bruising to the skin.

In an embodiment of the invention, the active ingredients from natural origins are selected from organic liquids of enzymes, herbal anti-inflammatories as well as topical analgesics. Examples of these ingredients are Pyrus Malus (apple) (enzymes), sesquiterpene lactone (arnica) (anti-inflammatory) and menthol, chamomile, ginger or Aloe Vera (topical analgesic), respectively.

In an additional aspect of the invention, the topical composition comprises additional ingredients selected, for example, from diluents, solvents, conditioning agents, fragrances, pH adjusters, emulsion agents, preservatives, antioxidants, emollients, surfactant agents, solubilizing agents, viscosity modifiers and chelating agents.

In another regard, the composition of present invention allows for very fast and efficient healing of hematomas or bruises on the skin, as well as relief of inflammation, pain and coloring change associated with these lesions.

In a preferred embodiment of the invention, the bruises or hematomas are the result of contusions and lesions to the tissue. It is important to mention that according to present invention the lesions to the skin do not involve those lesions where skin has been broken.

Additionally, the composition of present invention allows for the healing of bruises or hematomas which present themselves as part of the symptoms of illnesses such as for example, purpura, Henoch-Schonlein purpura, Senile Purpura or Bateman purpura, capillaritis, Von Willebrand disease, hemophilia, thrombocytopenia and venostasis among others. It must be highlighted that the topical composition of the invention does not provide a treatment for the healing or cure of these types of pathologies; it is merely for the relief of the bruises or hematomas associated with these illnesses, as well as for the relief of pain, inflammation and changes in coloring which are derived from hematomas.

In an embodiment of the invention, the topical composition allows for disaggregation of hematomas or bruises, decreasing the discoloration of the hematoma or bruise, lowering the pain associated with the hematoma or bruise and decreasing the inflammation generated by the hematomas derived from contusions, lesions to the tissue or illnesses. In an embodiment of the invention, the topical composition may be formulated as, for example, a cream, an ointment, an emulsion, a gel, a lotion, a paste, a bar, topical patches and aerosols, extracts or oils, preferably the topical composition is formulated as a cream or an ointment.

In an additional aspect, a method is provided to alleviate the symptoms associated with the contusion or lesion in tissues, particularly to disaggregate hematomas, lower the discoloration, lower the amount of pain associated with the hematoma or bruise and decrease inflammation, which comprises applying unto a human or animal subject in need of the same, an effective amount of the topical composition of the invention, particularly over the affected area.

In an embodiment of the invention a method to disaggregate the hematomas or bruises produced by illnesses is provided, as well as lowering discoloring, decreasing the amount of pain associated with the hematoma or bruise and decreasing the inflammation, which comprises applying unto a human or animal subject in need of the same, an effective amount of the topical composition of the invention, particularly on the affected area.

In another regard, the invention is directed at the use of the topical composition to ameliorate the symptoms caused by contusions or lesions on the tissues, particularly for the disaggregation of hematomas, lowering the discoloration of hematomas or bruises, decreasing the amount of pain associated with the hematoma or bruise and lowering inflammation.

In another embodiment, the invention is directed at the use of the topical composition to alleviate the hematomas and bruises produced by illnesses, particularly to disaggregate hematomas, lower the amount of discoloration of the hematomas or bruises, decrease the amount of pain associated with the hematoma or bruise and decrease inflammation.

### Brief Description of the Figures

The following figure helps to illustrate some preferred embodiments of the invention and is in no way limiting of the same.

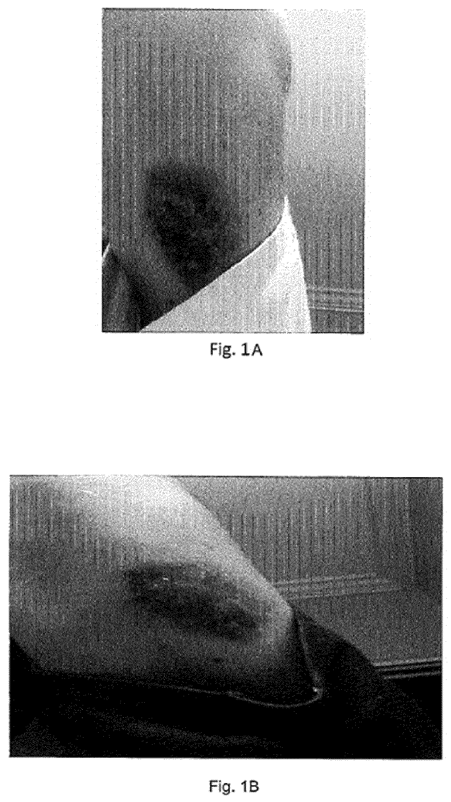

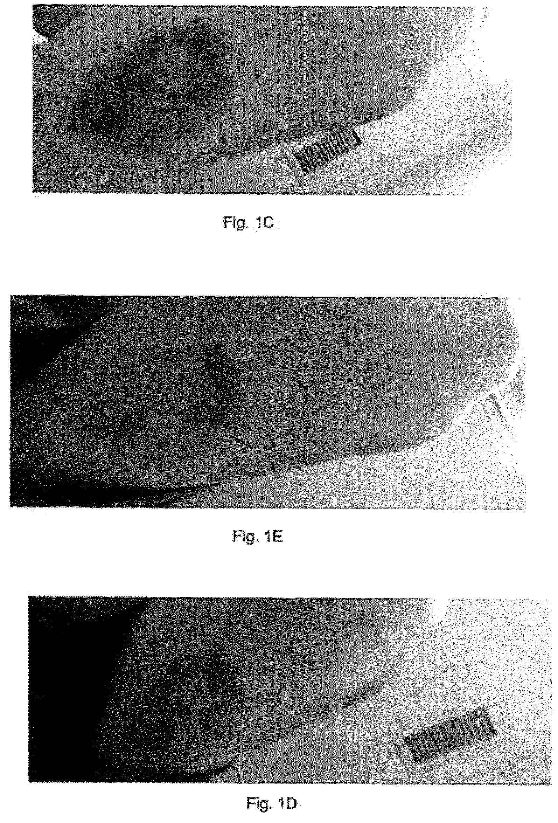

Figure 1A, B, C, D and E: show the effect of the topical composition applied unto a hematoma for a period of five days.

### Detailed Description of the Invention

The following description of technology is merely exemplary in nature of the subject matter, manufacture and use of one or more inventions, and is not intended to limit the scope, application, or uses of any specific invention claimed in this application or in such other applications as may be filed claiming priority to this application, or patents issuing therefrom. Regarding methods disclosed, the order of the steps presented is exemplary in nature, and thus, the order of the steps can be different in various embodiments. Except where otherwise expressly indicated, all numerical quantities in this description are to be understood as modified by the word "about" in describing the broadest scope of the technology.

The following definitions are provided to allow for a better comprehension of the invention:
The use of the term "approximately" provides an additional determined range. The term is defined in the following way. The additional range provided by the term is approximately ± 10%. By way of example, but not limitative, if it reads "approximately 40 cm", the exact range is between 36 to 44 centimeters.

The term "significantly", "significant" is geared to indicating a percentage of approximately 90-99%.

The terms "lower" or "decrease" according to the invention signify or comprise a percentage of approximately 90-99%. In other words, it must be understood that the decrease, for example of the coloring of the bruise involves a decrease of approximately 90-99% of said coloring.

The term "tissue" refers to the muscular and connective tissue. The lesions to the tissue involve sprains, distensions and slight contusions, where the presence of hematomas exists, as well as pain and inflammation and where the application of substances to the affected area is not contraindicated.

The term "lesion" is defined as the morphological or structural changes which some part of the body suffers from as a result of an internal or external damage. Within the lesions are the injuries to the skin produced by some trauma. The present invention is geared to non-grave or slight lesions, that is, those whose main symptomatology is centered in pain, inflammation and the presence of hematomas. For the purposes of present invention, skin is not broken after the lesion.

The term "contusion" is defined as a non-penetrating lesion on the body derived from the effect produced by a hard object. Present invention is oriented to non-grave or slight contusions with a symptomatology similar to that of the lesions according to that mentioned in the above lines. For terms of present invention, the skin is not broken as a result of the contusion.

The term "subject" encompasses animals or humans who present some lesion and/or contusion which are non-grave or an illness which produces bruises or hematomas as part of its symptoms.

The terms "healing" or "relief" in so far as it applies to this invention, must be understood as the decrease and/or elimination of the hematomas or bruises on the skin, as well as of the inflammation, pain and discolorations associated with it.

The term "preservative" is oriented towards substances which are added to the products, for example foods, pharmaceutical agents, paints, biological samples, wood, etc. in order to prevent decomposition originated by microbial growth or by undesired chemical changes. In a general manner, conservation may be achieved in two ways: chemical and physical. The present invention preferably is directed at chemical conservation methods, that is, to the application of chemical additives in the topical compositions of the invention which allow them to be conserved.

The chelating agent and chelation involves the formation or presence of two or more coordinated bonds separated between a polydented ligand (bonded in a multiple manner) and a simple central atom. Typically, these ligands are organic compounds and are called chelants, chelators, chelating agents or sequestering agents.

The term "emollient" or "moisturizer" refers to complex mixtures specially designed so that the outer layers of the skin (epidermis) be softer and more supple. This component increases the water content of the skin by decreasing the amount of evaporation. These types of components form part of the therapeutic commercial and cosmetic products. The topical composition of the invention may be formulated as, for example, a cream, an ointment, emulsion, gel, lotion, paste, stick, topical patches, aerosols, extracts or oils, preferably the topical composition is formulated as a cream or as an ointment.

In the case of the creams and ointments, these may be formulated with a water base or an oil base. A water base is preferred to ensure that the enzymes present in the composition do not become damaged or inactive, for example, Polyphenoloxidase (PPO), peroxidase (POD), among others, present in Pyrus malus.

The topical composition of the invention comprises as liquid active ingredients organic enzymes, herbal anti-inflammatories and topical analgesics. Examples of active ingredients used in the topical composition of the invention are Pyrus malus (apple) (enzymes), Sesquiterpene lactone (Arnica) (anti-inflammatory) and menthol, chamomile (chamomile), Ginger and Aloe Vera (topical analgesics), respectively.

The topical composition of the invention may comprise additional ingredients selected from, for example, diluents, solvents, conditioning agents, fragrance, pH adjusters, emulsifiers, preservatives, antioxidants, emollients, surfactant agents, solubilizing agents, viscosity modifiers and chelating agents.

Water may be used as a diluent or solvent. Additionally, polar organic diluents can be used, selected from, for example, alcohols and glycols. The preferred diluent or solvent is water.

The Carbapol polymer (acrylates/C₁₀₋₃₀ alkyl acrylate Crosspolymer) is an example of the alcohols and glycols which may be used in present invention.

Examples of conditioning agents and emollient agents are cetyl alcohol, propylene glycol, isopropyl palmitate, Chamomilla Recutita (Matricaria) Flower Extract, sodium hyaluronate, tocopheryl acetate, cetearyl ethylhexanoate, methyl gluceth-20, Sweet Almond Oil (Prunus Amygdalus Dulcis oil (sweet almond)),shea butter (Butyrospermum Parkii), cocoa butter ((Butyrospermum Parkii), Broccoli Oil (Brassica Seed Oleracea Italica Oil), caprylic/capric triglyceride, Kokum butter (Garcinia indica Seed Butter), hydrogenated castor oil, jojoba oil (Simmondsia Chinensis Seed Oil), ethylhexyl Olivato, stearyl alcohol (also called N-octadecanol), etc.

The organic liquid with enzymes comprises Pyrus malus. In one embodiment the organic liquid with enzymes comprises the enzymes present in Pyrus malus, for example, Polyphenol oxidase (PPO), peroxidase (POD), among others.

In a preferred embodiment, the organic liquid comprises the Pyrus Malus and Arnica combination.

The anti-inflammatories for the composition of the invention are selected, for example, from among: Sesquiterpene lactone (Arnica), Calendula (Calendula officinalis), ginger (Zingiber officinale), thymol, evening primrose oil (Oenothera biennis), Flaxseed (Linum usitatissium), Violet (Viola odorata), sage (Salvia officinalis), etc. preferably the anti-inflammatory is arnica.

The topical analgesics are selected from among, for example, menthol, Chamomile (Chamomila), ginger or Aloe Vera.

As examples of the fragrances which may be used in the composition are for example, menthol, Glyoxal, stearyl alcohol, corn oil (Zea Mays).

It is important to mention that given the inherent smell which menthol exerts, this component may also be used as a fragrance as it provides a pleasant smell of the composition of present invention to the subject.

To avoid irritation to the skin of the subject the composition of the invention presents neutral parameters. The pH adjusters which may be used can be selected from among, for example, triethanolamine and others known in the art.

The emulsion agents and other surfactant agents for the composition of present invention may be selected from among, for example, glyceryl stearate, PEG-100 stearate, Polysorbate 80 (Polyoxyethylene(20)sorbitanmonooleate); Polysorbate 20 (polyoxyethylene(20)sorbitanmonolaurate); PEG/PPG -11/21 Copolymer; Hydrogenated castor oil PEG-40; Cetyl alcohol; Palmitic acid; corn oil (Zea mays); lecithin; Stearyl alcohol; PEG/PPG-128/54 Copolymer; Glyceryl oleate, etc.

As an example of the preservatives which may be used in the composition of present invention are: phenoxyethanol; a mixture of phenoxyethanol, benzoic acid and Dehydroacetic acid; a mixture of DMDM Hydantoin and lodopropinilo butylcarbamate; parabens selected from, for example, butylparaben, ethylparaben, methylparaben, Propylparaben; glyoxal; Diazolidinyl Urea; Imidazolidinyl urea; a mixture of Methylisothiazolinone and ethylhexylglycerin and water; A mixture of Butylene Glycol Propionate and Undecylenic Glycerides (and) Sodium Usnate; P-hydroxybenzoic acid. The anti-oxidants may be selected from among, for example tocopheryl acetate; Carnosine (beta-alanyl-L- histidine), Resveratrol (5-[(E)-2-(4-hydroxyphenyl) ethenyl] benzene-1, 3-diol); Silver Ear Mushroom Extract (Tremella fuciformis extract); PhytoCare-HA® (Silver Ear Mushroom Extract (Tremella fuciformis)); Superoxide dismutase (SOD).

The solubilizing agents may be selected from among, for example, Polisorbate 80. The solubilizing agents may also be used as surfactant agents in the topical composition of the invention.

The viscosity modifying agents may be selected from among, for example, Acrylates/C1-30 alkyl acrylate Crosspolymer (Carbapol Polymer).

The chelating agents used in the topical compositions of the present invention can be selected from among, for example, tetrasodium EDTA; Citric acid; Glucono-delta-lactone or Gluconolactone ((3R, 4S, 5S, 6R)-3,4,5- trihydroxy-6-(hydroxymethyl)tetrahydro-2H-pyran-2-one); Cyclohexanediamine tetraacetic acid; Carnosine (beta-alanyl-L-histidine), Resveratrol (5-[(E)-2-(4-hydroxyphenyl)ethenyl]benzen-1,3-diol); Silver Ear Mushroom Extract (Tremella fuciformis extract), etc.

The composition of the invention can comprise excipients, which may be selected from among, for example, water, glycerine, C1-4 alcohols, fatty alcohols, fatty ethers, fatty esters, polyols, glycols, vegetable oils, mineral oils, liposomes, laminar lipid materials, silicone oils and combinations thereof.

The active as well as the additional ingredients which are comprised in the topical composition of the invention may be found in concentrations from approximately 1% to approximately 80%, preferably from approximately 2% up to approximately 70%, and even more preferably from approximately 20% up to approximately 65%.

The cream, ointment, emulsion, gel, lotion, paste, bar stick, topical patches, aerosols, extracts or oils which are prepared from the topical composition of the invention may be applied directly over the affected area of the skin of the subject to be healed from the bruises or hematomas resulting from contusions, lesions to the tissue or from illnesses. The compositions of the invention particularly allow the hematomas to disaggregate and to decrease the discoloring of the hematomas or bruises, the pain and the inflammation associated with the hematoma.

A method according to the invention for disaggregating hematomas; decreasing discoloring; lowering the pain associated with the hematoma or bruise and decreasing the inflammation, comprises applying over the affected area of a subject an effective amount of the topical composition of the invention.

The application periods for the composition of the invention as well as the amount to be applied will be in function of the severity of the lesion to the skin, the size of the hematoma, as well as the general state of health of the subject. As would be obvious to a person skilled in the art, the larger the hematoma ends up being or the subject presenting the hematoma being of an advanced age, the greater the healing time shall be.

It should be highlighted that the hematomas or bruises are transitory afflictions and the application of the composition shall only be for the time in which the hematoma is present.

In one embodiment of the invention, the topical composition may be applied liberally over the area to be treated and reapplied according to the need after its absorption into the skin and while the bruises or hematomas continue to be present on the skin.

In another embodiment of the invention a layer of topical composition may be applied unto the affected area at least 01 time per day, preferably at least 02 times per day until the disappearance of the bruise or hematoma.

In another embodiment of the invention a layer of topical composition may be applied unto the affected area at least 02 times per day until the disappearance of the bruise or hematoma.

The healing time for the bruises or hematomas upon application of the topical composition of the invention is in function of the perfusion of the subject, same which generally varies with the age of the subject, his/her nutritional state, the application frequency and the subject's general state of health.

In one embodiment of the invention, in a time period of 3 to 6 days, preferably 3 to 5 days, the hematomas are disaggregated; the discoloration is significantly decreased or disappears; the pain associated with the hematoma or bruise is significantly decreased or disappears; and the inflammation is diminished.

### PREPARATION METHOD

The topical composition of the invention is preferably prepared as an aqueous emulsion. Particularly, some active ingredients are subjected to a fermentation process and others to an infusion process. Once a concentrated active ingredient is obtained; this is homogenized and stabilized in the final formulation. For example, the active ingredient Pyrus Malus (apple) is subjected to a fermentation process and the arnica infusion is concentrated more as an anti-inflammatory agent.

### EXAMPLES

The following illustrates an embodiment of the topical composition of the invention:

| Ingredients | Purpose |
|---|---|
| Water | Diluent/Dissolvent |
| Propylene glycol | Conditioning Agent |
| Pyrus Malus (Apple) | Organic liquid with enzymes |
| sesquiterpene lactone (Arnica) | Anti-inflammatory |
| Menthol | Fragrance and Analgesic |
| Isopropyl palmitate | Conditioning Agent |
| Triethanolamine | pH Adjuster |
| Glyceryl stearate | Surfactant Agent |
| PEG -100 stearate | Surfactant Agent |
| Phenoxyethanol | Preservative |
| Chamomilla Recutita (Matricaria) Flower Extract | Conditioning Agent |
| Sodium hyaluronate | Conditioning Agent |
| Tocopheryl acetate | Conditioning Agent, Antioxidant |
| Cetearyl ethylhexanoate | Conditioning Agent |
| Methyl gluceth - 20 | Conditioning Agent |
| Cetyl alcohol | Emollient |
| Polysorbate 80 | Surfactant Agent/Solubilizing Agent |
| Carbapol polymer (acrylates / C10-30 alkyl acrylate Crosspolymer) | Viscosity modifying agent |
| EDTA tetrasodium | Chelating Agent |

### Example of the Application:

For a 46 year old male subject with large bruises covering an approximate area of 14*10cm on the left arm, the topical composition of the invention was applied for a period of five days. The application was undertaken once per day, using a sufficient amount to cover the affected area.

From the first day of application a decrease in the size of the bruise was noted. The decrease in size for the bruise was progressive the second day, the third day and successively during the application time period. On the sixth day, the bruise had completely disappeared and application was discontinued (see Figure 1).

Finally, the embodiments previously described are merely illustrative and not limitative in terms of the scope of present invention, to which full extension of the attached claims should be granted, in addition to all and any equivalent of the same. As would be obvious to persons skilled in the art the variations and changes to the present invention can be accomplished without deviating from the summarized concepts of the above description. It is considered that these changes are included to lie within the claimed scope of present invention.

## Claims

1. A topical composition comprising: an organic liquid with enzymes, an anti-inflammatory and an analgesic.

2. The topical composition according to claim 1, wherein the composition additionally includes a member selected from the group consisting of: diluents, solvents, conditioning agents, fragrance, pH adjusters, emulsion agents, preservatives, antioxidants, emollients, surfactant agents, solubilizing agents, viscosity modifiers, chelating agents, and combinations thereof.

3. The topical composition according to claim 1, wherein:
the organic liquid with enzymes includes a member selected from the group consisting of Pyrus Malus, Sesquiterpene lactone (Arnica), Calendula (Calendula officinalis), ginger (Zingiber officinale), thymol, oil of evening primrose (Oenothera biennis), Flaxseed (Linum usitatissium), Violet (Viola odorata), sage (Salvia officinalis), and combinations thereof; and
the analgesic includes a member selected from the group consisting of menthol, chamomile (chamomila), ginger, Aloe Vera, and combinations thereof.

4. The topical composition according to claim 1, wherein the organic liquid with enzymes comprises a combination of Pyrus malus and arnica.

5. The composition according to claim 1, wherein the composition is formulated as a cream, an ointment, emulsion, gel, lotion, paste, bar stick, topical patche, aerosol, extract, or oil.

6. The topical composition according to claim 5, wherein the composition is formulated as a cream or an ointment.

7. The topical composition according to claim 2, comprising concentrations of the organic liquid with enzymes, the anti-inflammatory, and the analgesic from approximately 1% up to approximately 80%.

8. A method to heal an affected area of a subject, the affected area having a bruise or a hematoma derived from a contusion, lesion to the skin, or as a result of illness, the method comprising applying over the affected area of the subject an effective amount of the topical composition as claimed in claim 1.

9. The method of claim 8, wherein the healing comprises a member selected from the group consisting of disaggregating the hematoma or the bruise, decreasing the discoloration of the hematoma or the bruise, decreasing the pain associated with the hematoma or the bruise, lowering inflammation at the affected area, and combinations thereof.

10. The method of claim 8, wherein the topical composition is formulated as a cream, an ointment, emulsion, gel, lotion, paste, bar stick, topical patche, aerosol, extract, or oil.

11. The method of claim 10, wherein the topical composition is formulated as a cream or an ointment.

12. The method of claim 8, wherein the lesion to the skin is the result of a contusion or a lesion on a tissue of the subject.

13. The method of claim 8, wherein the illness is purpura, Henoch-Schonlein purpura, Senile Purpura or Bateman purpura, capillaritis, Von Willebrand disease, hemophilia, thrombocytopenia, or venostasis.

14. The method of claim 9, wherein the topical formulation is applied at least one time per day unto the affected area.

15. The method of claim 9, wherein the topical formulation is applied liberally unto the affected area.
